# EUROPÄISCHE PATENTSCHRIFT

(11) **EP 0 657 141 B1**
(45) Veröffentlichungstag und Bekanntmachung des Hinweises auf die Patenterteilung: **04.02.1998**
(21) Anmeldenummer: 94117489.8
(22) Anmeldetag: 05.11.1994
(51) Int. Cl.: A61B 17/36, A61N 5/06

(54) **Vorrichtung für die interstitielle thermotherapeutische Behandlung**
Apparatus for institial thermotherapeutical treatment
Appareil pour le traitement thermothérapeutique du tissue interstitiel

(30) Priorität: 09.12.1993 DE 4341967
(43) Veröffentlichungstag der Anmeldung: 14.06.1995
(73) Patentinhaber: Dornier Medizintechnik GmbH, D-82110 Germering (DE)
(72) Erfinder: Hessel, Stefan, Dr., D-81927 München (DE); Frank, Frank, Dr., D-85560 Ebersberg (DE); Hauptmann, Gerhard, D-81827 München (DE); Hiereth, Werner, D-81825 München (DE)
(74) Vertreter: Frick, Gerhard, Dipl.-Ing.

(56) Entgegenhaltungen:
- EP-A- 0 335 022
- WO-A-84/04879
- DE-A- 3 934 646
- US-A- 4 316 467

## Beschreibung

Die Erfindung betrifft eine Vorrichtung für die interstitielle thermotherapeutische Behandlung von biologlschem Gewebe umfassend einen Dauerstrichlaser , insbesondere einen Nd:YAG Laser, mit einem Lichtleiter , über welchen innerhalb eines Bestrahlungszeitraumes Laserstrahlung in das zu behandelnde Gewebe zur Erzeugung einer Gewebsnekrose führbar ist.

Bei der von Muschter und Hofstetter in "Münch.med.Wschr. 134(1992) Nr. 40, S. 630-634 beschriebenen interstitiellen Thermotherapie mit Laserstrahlung wird ein speziell ausgeführter Lichtleiter (z.B. gemäß EP 0 292 695 B1) in das zu erwärmende Gewebe eingeführt. Die Applikation der Laserstrahlung bewirkt dabei eine thermische Denatuierung des Gewebes und damit die Bildung einer Koagulationsnekrose. Die Einwirkung der applizierten Laserstrahlung wird zum einen von den Laserparametern wie Wellenlänge, Bestrahlungsdauer, Laserleistung und Leistungsdichte bestimmt und zum anderen von den Gewebeparametern wie Absorptionsvermögen, Streuung, Wärmeleitung, Gewebestruktur und Durchblutung.

Bei Temperaturen über 60° ändern sich die Gewebeparameter, z.B. durch Koagulation, Karbonisation oder durch Verschluß der Blutgefäße. Insbesondere die Gewebekarbonisation, z.B. durch zu hohe Laserleistung oder durch zu hohe Leistungsdichte, führt zu einer drastischen Steigerung des Absorptionsvermögens, wodurch ein tiefes Eindringen der Photonen in das Gewebe verhindert wird. Eine Karbonisation des Gewebes in unmittelbarer Umgebung des Lichtleiters führt außerdem zu einer schlagartig erhöhten thermischen Belastung des Lichtleiters und kann damit dessen Zerstörung bewirken. Aus diesem Grund wurde bisher die Laserleistung auf einen weit unterhalb der Zerstörungsschwelle für den Lichtleiter gelegenen Wert eingestellt, so daß einerseits sichergestellt wurde, daß weder das Gewebe während des Bestrahlungszeitraumes karbonisiert noch der Lichtleiter überlastet wird, andererseits jedoch eine Koagulationsnekrose mit ausreichender Größe entsteht.

Dies hat jedoch z.B. bei der Behandlung der benignen Prostatahyperplasie zu Bestrahlungszelträumen pro Punktion von jeweils 10 Minuten geführt, wobei durchschnittlich für eine Behandlung 8 Punktionen notwendig sind. Damit ergibt sich bei dem bekannten Verfahren eine Gesamtbehandlungsdauer von ca. 2 Stunden.

Verfahren und Vorrichtungen zur Regelung der Leistung von medizinischen Lasergeräten sind vielfach bekannt. So beschreiben die US 4 316 467 oder die DE 39 34 646 Regelungen für einen solchen Laser, bei welchen die von einem bestrahlten Gewebe reemittierte Strahlung auf einen Detektor gelangt und dessen Ausgangssignal als Steuersignal für eine Änderung der Strahlungsleistung des Lasers benutzt wird. Bei der aus obigen Schriften bekannten Art der Laserbestrahlung finden Gewebeänderungen und damit Änderungen der reemittierten Strahlung zuerst an der Gewebeoberfläche statt, so daß schon geringste Karbonisationsprozesse der Gewebeoberfläche zu einer Leistungsreduzierung bzw. Beendigung der Bestrahlung führen.

Es ist Aufgabe der vorliegenden Erfindung, eine Vorrichtung für die interstitielle thermotherapeutische Behandlung von biologischem Gewebe zu schaffen, welche unter Beibehaltung der o.g. Randbedingungen, insbesondere der Vermeidung einer Zerstörung des Lichtleiters, eine erheblich kürzere Behandlungsdauer ermöglicht.

Diese Aufgabe wird durch eine Vorrichtung mit den Merkmalen des Patentanspruchs 1 gelöst.

Die Erfindung berücksichtigt den speziell bei der interstitiellen Behandlung mit Lichtleitern auftretenden, komplexen Vorgang der Wärmeleitung am Übergang zwischen Lichtleiter und Gewebe durch eine gesteuerte kontinuierliche oder stufenweise Reduzierung der Laserleistung während eines Bestrahlungszeitraumes nach dem Einstich des Lichtleiters in das Gewebe. Bei dem anfänglich kalten Gewebe kann kurzzeitig eine sehr viel höhere Laserleistung appliziert werden, bis die Karbonisationstemperatur erreicht ist. Da mit der Aufheizung des Gewebes über die applizierte Laserstrahlung gleichzeitig auch ein Wärmeabfluß über das umgebende Gewebe erfolgt, welcher jedoch bei zunehmender Erwärmung ständig geringer wird, wird die Laserleistung während eines Bestrahlungszeitraumes kontinuierlich oder stufenweise von dem anfänglich nur durch die maximale Leistung des Lasers bzw. der Zerstörungsschwelle der lichtleitenden Komponenten begrenzten Wert so reduziert, daß die Karbonisationstemperatur des Gewebes innerhalb des Bestrahlungszeitraumes nie überschritten wird. Damit verkürzt sich die Bestrahlungsdauer für die Erzeugung einer gleich großen Koagulationsnekrose etwa auf die Hälfte der bisherigen Bestrahlungszeit.

Die gesteuert ablaufende Leistungsreduzierung des Lasers läßt sich im Gegensatz zu regelungstechnischen Maßnahmen problemlos mit den bekannten für medizinische Anwendungen vorgesehenen Lasern realisieren.

Weiterhin bewirkt die erfindungsgemäße Vorrichtung, daß in der Umgebung des eingesetzten Lichtleiters befindliche Blutgefäße erheblich schneller verschlossen werden und damit eine deutliche Verringerung des Wärmeabtransportes durch Konvektion erzielt wird. Auch die Koagulationstemperatur am Übergang zwischen Lichtleiter und Gewebe wird erheblich schneller erreicht.

Die optimale Steuerkurve für die Reduzierung der Laserleistung ist vor allem gewebeabhängig und wird vorteilhafterweise empirisch ermittelt. Für die meisten Behandlungsfälle hat sich eine Laserleistung von anfänglich maximal 25 Watt mit laufender Reduzierung während des Bestrahlungszeitraumes auf minimal 5 Watt als brauchbar erwiesen.

Die Erfindung wird im folgenden anhand eines schematisch dargestellten Ausführungsbeispieles näher beschrieben:

An einen für die medizinische Anwendung geeigneten Nd:YAG-Laser in der Leistungsklasse von 40 Watt mit integrierter Leistungsregelung 1.1 ist eine Lichtleitfaser 2 angeschlossen, deren distales Ende mit einer zirkumferenziell abstrahlenden Spitze 2.1 für eine interstitielle thermotherapeutische Behandlung versehen ist. Diese Spitze 2.1 wird in das zu behandelnde Gewebe zur Erzeugung einer Koagulationsnekrose eingestochen. Die Leistungsregelung 1.1 des Lasers wird über eine Zeitablaufsteuerung 3 angesteuert, welche im gezeigten Ausführungsbeispiel eine stufenweise Leistungsreduzierung des Lasers erzeugt. Die Zeitablaufsteuerung 3 wird über einen Startschalter 4 eingeschaltet.

Mit Hilfe der Zeitablaufsteuerung 3 werden die Dauer eines Bestrahlungszeitraumes, die Anfangs- und Endleistung P des Lasers sowie die Steuerkurve (gestuft oder kontinuierlich) festgelegt.

Bei einem in-vitro-Versuch an Lebergewebe wurden vergleichende interstitielle Behandlungen mit herkömmlicher, konstanter Laserleistung und mit erfindungsgemäß reduzierter Laserleistung während eines Bestrahlungszeitraumes durchgeführt. Die Ergebnisse zeigen, daß mit dem erfindungsgemäßen Bestrahlungskonzept zum einen eine deutlich geringere Gesamtenergie für eine vorgegebene Koagulationsgröße notwendig ist als bei Verwendung konstanter Laserleistung und weiterhin eine Reduzierung der Bestrahlungsdauer auf die Hälfte erzielt wurde. Während bei konstanter Laserleistung über einen Bestrahlungszeitraum von 10 Minuten die Leistung auf etwa 7 Watt beschränkt bleiben muß, wobei mit einer applizierten Gesamtenergie von 4200 J eine Koagulationsnekrose in Form eines Ellipsoides mit den Achsen 18 x 24 mm erzeugt wurde, wird bei dem erfindungsgemäßen Bestrahlungskonzept die Laserleistung von anfänglich 20 Watt stufenweise auf 7 Watt reduziert, wobei bei einer Bestrahlungsdauer von 5 Minuten eine Gesamtenergie von 2820 J appliziert wird und eine Koagulationsnekrose der Größe 21 x 29 mm entsteht.

Bei diesen in-vitro-Versuchen wurde die Laserleistung nur relativ grob gestuft reduziert, so daß mit einer kontinuierlichen, optimierten Steuerkurve noch eine weitere Verbesserung des Behandlungsergebnisses zu erwarten ist.

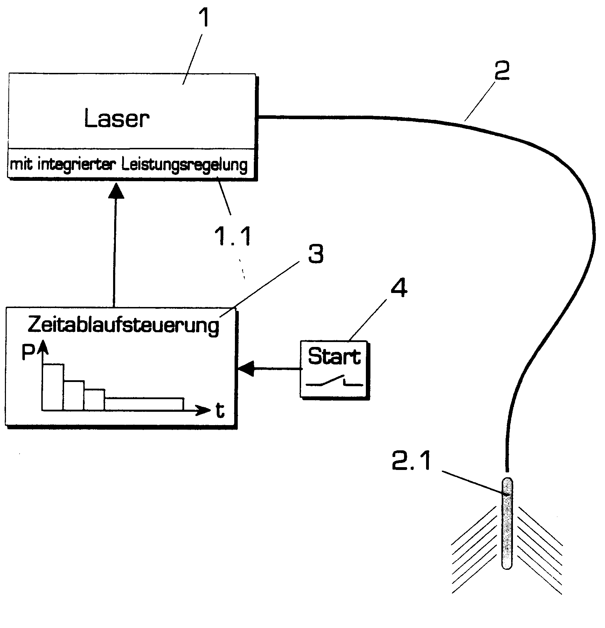

## Patentansprüche

1. Vorrichtung für die interstitielle thermotherapeutische Behandlung von biologischem Gewebe umfassend einen Dauerstrichlaser (1), insbesondere einen Nd:YAG Laser, mit einem Lichtleiter (2), über welchen innerhalb eines Bestrahlungszeitraumes Laserstrahlung in das zu behandelnde Gewebe zur Erzeugung einer Gewebsnekrose führbar ist, **dadurch gekennzeichnet,** daß Mittel (3) vorhanden sind, die die Strahlungsleistung des Lasers während des Behandlungszeitraumes zeltgesteuert kontinuierlich oder stufenweise so reduzieren, daß die Karbonisationstemperatur des Gewebes innerhalb des Behandlungszeitraumes nicht überschritten wird.

2. Vorrichtung nach Anspruch 1, **dadurch gekennzeichnet,** daß Mittel (3) vorhanden sind, die die Strahlungsleistung des Lasers (1) während des Bestrahlungszeitraumes kontinuierlich oder stufenweise derart reduzieren, daß die Temperatur innerhalb einer Koagulationsnekrose geringfügig unterhalb der Karbonisationstemperatur des bestrahlten Gewebes bleibt.

3. Vorrichtung nach Anspruch 1 oder 2, **dadurch gekennzeichnet,** daß die Mittel (3) die anfängliche Strahlungsleistung des Lasers (1) Innerhalb eines Bestrahlungszeitraumes in Abhängigkeit von dem Zerstörungsschwellwert für die Lichtleitfaser (2) einstellen.

4. Vorrichtung nach einem der Ansprüche 1 bis 3, **dadurch gekennzeichnet,** daß Mittel (3) zur Reduzierung der Strahlungsleistung des Lasers (1) innerhalb eines Bestrahlungszeitraumes mit 1/t³ vorhanden sind, wobei t die Zeit darstellt.

5. Vorrichtung nach einem der Ansprüche 1 bis 4, **dadurch gekennzeichnet,** daß Mittel (3) vorhanden sind, die die Strahlungsleistung des Lasers (1) während eines Bestrahlungszeitraumes kontinuierlich oder stufenweise von maximal 25 Watt zu Beginn des Bestrahlungszeitraumes auf minimal 5 Watt am Ende des Bestrahlungszeitraumes reduzieren.

## Claims

1. An apparatus for the interstitial thermo-therapeutic treatment of biological tissue, comprising a continuous-wave laser (1), particularly a Nd:YAG laser, with an optical waveguide (2) via which laser radiation can be led into the tissue to be treated over an irradiation period in order to produce a tissue necrosis, **characterised in that** means (3) are present which reduce the radiation output of the laser continuously or stepwise during the treatment period, in a manner which is controlled with respect to time, so that the carbonisation temperature of the tissue is not exceeded within the treatment period.

2. An apparatus according to claim 1, **characterised in that** means (3) are present which reduce the radiation output of the laser (1) continuously or stepwise during the treatment period so that the temperature inside a coagulation necrosis remains slightly below the carbonisation temperature of the irradiated tissue.

3. An apparatus according to claim 1 or 2, **characterised in that** the means (3) adjust the initial radiation output of the laser (1) within a treatment period depending on the destruction threshold for the optical waveguide fibres (2).

4. An apparatus according to any one of claims 1 to 3, **characterised in that** means (3) are present for reducing the radiation output of the laser (1) within an irradiation period according to 1/t³, wherein t denotes time.

5. An apparatus according to any one of claims 1 to 4, **characterised in that** means (3) are present which reduce the radiation output of the laser (1) continuously or stepwise during a treatment period from a maximum of 25 watts at the start of the treatment period to a minimum of 5 watts at the end of the treatment period.

## Revendications

1. Dispositif pour le traitement thermothérapeutique interstitiel de tissu biologique comprenant un laser continu (1), notamment un laser Nd:YAG, avec une fibre optique (2) à l'aide de laquelle, à l'intérieur d'un temps d'irradiation, un rayonnement laser peut être amené dans le tissu à traiter aux fins de produire une nécrose du tissu, caractérisé par le fait que des moyens (3) sont prévus qui diminuent progressivement ou par paliers, en fonction du temps, la puissance émise du laser pendant le temps de traitement de telle sorte que la température de carbonisation du tissu ne soit pas dépassée à l'intérieur du temps de traitement.

2. Dispositif selon la revendication 1, caractérisé par le fait qu'il est prévu des moyens (3) qui diminuent progressivement ou par paliers la puissance émise du laser (1) pendant le temps d'irradiation, de telle sorte que la température dans une nécrose par coagulation reste légèrement en-dessous de la température de carbonisation du tissu traité.

3. Dispositif selon la revendication 1 ou 2, caractérisé par le fait que les moyens (3) règlent la puissance de début du laser (1) à l'intérieur d'un temps d'irradiation en fonction du seuil de destruction de la fibre optique (2).

4. Dispositif selon une des revendications 1 à 3, caractérisé par le fait que des moyens (3) pour diminuer la puissance émise du laser (1) à l'intérieur d'un temps d'irradiation avec 1/t³ sont prévus, t représentant le temps.

5. Dispositif selon une des revendications 1 à 4, caractérisé par le fait qu'il est prévu des moyens (3) qui diminuent progressivement ou par paliers la puissance émise du laser (1) à l'intérieur d'un temps d'irradiation depuis 25 watts maximum, au début du temps d'irradiation, jusqu'à 5 watts minimum, à la fin du temps d'irradiation.
